# EUROPÄISCHE PATENTSCHRIFT

(11) **EP 2 956 073 B1**
(45) Veröffentlichungstag und Bekanntmachung des Hinweises auf die Patenterteilung: **04.01.2017**
(21) Anmeldenummer: 14704569.4
(22) Anmeldetag: 13.02.2014
(51) Int. Cl.: A61B 17/70, A61B 17/80, A61B 17/86

(54) **KNOCHENPLATTEN-SYSTEM**
BONE PLATE SYSTEM
SYSTÈME DE PLAQUES OSSEUSES

(30) Priorität: 14.02.2013 EP 13155242
(43) Veröffentlichungstag der Anmeldung: 23.12.2015
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2014/052787
(87) Internationale Veröffentlichungsnummer: WO 2014/125005

(56) Entgegenhaltungen:
- EP-A2- 0 242 842
- US-A- 5 397 363
- US-A1- 2006 122 605
- US-A1- 2010 057 134
- US-A1- 2011 118 784
- US-B1- 6 406 478

## Beschreibung

Die Erfindung betrifft ein Knochenplatten-System mit einer Knochenplatte, mit einer Druckplatte und mit einer Knochenschraube. Die Knochenplatte umfasst ein Durchgangsloch mit einer winkelvariablen Sitzfläche für die Knochenschraube. In einem verbundenen Zustand des Systems ist die Knochenschraube in das Durchgangsloch eingesetzt und der Kopf der Knochenschraube zwischen der Knochenplatte und der Druckplatte eingeschlossen. Zum Befestigen der Druckplatte an der Knochenplatte ist eine zwei Befestigungsschrauben umfassende Schraubverbindung vorgesehen.

Knochenplatten dieser Art werden am Knochen fixiert, indem die Knochenschraube durch das Durchgangsloch hindurchgeführt wird und in das Knochenmaterial eingeschraubt wird. Angewendet werden die Knochenplatten beispielsweise, um Knochen oder Knochenfragmente relativ zueinander zu fixieren. Möglich sind auch andere Anwendungsfälle, bei denen die Knochenplatte beispielsweise ein Element einer Prothese, wie etwa einer Gelenkprothese ist.

Indem die Sitzfläche des Durchgangslochs so gestaltet ist, dass sie die Knochenschraube unter unterschiedlichen Winkeln aufnehmen kann, kann der Chirurg während der Operation den Winkel, unter dem er die Knochenschraube einschraubt, innerhalb gewisser Grenzen frei wählen. Er kann die Knochenschraube also so einsetzen, wie es den anatomischen Gegebenheiten am besten entspricht.

Die Druckplatte wird anschließend so mit der Knochenplatte verbunden, dass der Kopf der Knochenschraube zwischen der Druckplatte und der Knochenplatte eingeklemmt wird. Die Knochenschraube wird dadurch unter den gewählten Winkel relativ zu der Knochenplatte fixiert. Die Winkelstabilität zwischen der Knochenschraube und der Knochenplatte hat zur Folge, dass zwischen der Knochenschraube und dem Knochenmaterial geringere Lasten pro Flächeneinheit übertragen werden, wodurch das Knochenmaterial geschont wird. Ein Knochenplatten-System dieser Art ist beispielsweise aus EP 0 201 024 A1 bekannt.

Der Erfindung liegt die Aufgabe zu Grunde, ein Knochenplatten-System vorzustellen, bei dem die Knochenschraube mit hoher Zuverlässigkeit fixierbar ist. Ausgehend vom genannten Stand der Technik wird die Aufgabe gelöst mit den Merkmalen des Anspruchs 1. Erfindungsgemäß sind die Achsen der Befestigungsschrauben gespreizt, so dass der Abstand zwischen den Achsen der Befestigungsschrauben in Einschraubrichtung größer wird.

Durch die erfindungsgemäße Spreizung der Achsen wird erreicht, dass die Druckplatte im verbundenen Zustand unter einer Zugspannung steht, die in einer zu der Ebene der Knochenplatte parallelen Richtung wirkt. Durch diese Spannung wird der Knochenschraube zusätzlicher Halt verliehen. Die Knochenschraube ist also mit erhöhter Zuverlässigkeit relativ zu der Knochenplatte fixiert.

Das erfindungsgemäße Knochenplatten-System kann so gestaltet sein, dass die Druckplatte durch den Kopf der in die Knochenplatte eingesetzten Knochenschraube auf Abstand von der Knochenplatte gehalten wird, wenn die Druckplatte in entspanntem Zustand ist. Dies bedeutet, dass die entspannte Druckplatte nicht flächig auf der Knochenplatte aufliegen kann, wenn der Kopf der Knochenschraube zwischen der Knochenplatte und der Druckplatte eingeschlossen ist.

Zum Herstellen der Schraubverbindung zwischen der Druckplatte und der Knochenplatte werden die Befestigungsschrauben jeweils durch eine Aufnahmeöffnung der Druckplatte hindurchgeführt und in eine Bohrung der Knochenplatte eingeführt. Damit die Befestigungsschrauben leicht in Eingriff gebracht werden können, ist es von Vorteil, wenn ein erstes Aufnahmeloch der Druckplatte mit einer ersten Bohrung der Knochenplatte fluchtet und ein zweites Aufnahmeloch der Druckplatte mit einer zweiten Bohrung der Knochenplatte fluchtet, wenn die Druckplatte entspannt ist. Vorzugsweise ist dies der Fall ist, wenn die entspannte Druckplatte einen Abstand zu der Knochenplatte hat. Die Druckplatte kann dann auf den Kopf der Knochenschraube aufgesetzt werden und beide Befestigungsschrauben können ohne Spannung in Eingriff gebracht werden.

Unter Spannung wird die Druckplatte erst mit dem Anziehen der Befestigungsschrauben gesetzt. Dies hat einerseits zur Folge, dass die Enden der Druckplatte näher an die Knochenplatte herangezogen werden, so dass die Druckplatte im zusammengesetzten Zustand des Knochenplatten-Systems bogenförmig verformt ist, also unter eine Biegespannung steht. Zum anderen wird die Druckplatte nach außen gespannt, so dass die Druckplatte im zusammengesetzten Zustand des Knochenplatten-Systems unter einer Zugspannung steht. Beides trägt dazu bei, den Kopf der Knochenschraube sicher zwischen der Druckplatte und der Knochenplatte zu fixieren.

Mit dem Durchbiegen der Druckplatte verändert sich der Winkel zur Befestigungsschraube. Da Spannungen zwischen dem Kopf der Befestigungsschraube und der Druckplatte unerwünscht sind, ist es von Vorteil, wenn zwischen dem Kopf der Befestigungsschraube eine winkelvariabele Aufnahme vorgesehen ist. Das erste Aufnahmeloch und/oder das zweite Aufnahmeloch der Druckplatte können deswegen eine winkelvariabele Sitzfläche für die Befestigungsschraube haben. Beispielsweise kann die Sitzfläche sphärisch geformt sein und der Kopf der Befestigungsschraube eine daran angepasste Form haben.

Der Winkel, den die Achsen der Befestigungsschraube miteinander einschließen, kann beispielsweise zwischen 5° und 30°, vorzugsweise zwischen 10° und 20° liegen. Versuche haben gezeigt, dass auch mit einem größeren Winkel gute Ergebnisse erzielt werden. Der Winkel kann beispielsweise auch zwischen 30° und 90° liegen.

Die für die Befestigungsschrauben bestimmten Bohrungen in der Knochenplatte können als Sackbohrung oder als durchgehende Bohrung ausgebildet sein. Die Bohrung kann ein Innengewinde aufweisen, in das die Befestigungsschraube eingeschraubt werden kann. Vorzugsweise schließen die Bohrungen eines oder mehrere der für die Knochenschrauben bestimmten Durchgangslöcher zwischen sich ein. Die Achse der Bohrung schließt mit einer Tangentialebene, die im Bereich der Bohrung an die Knochenplatte angelegt ist, vorzugsweise einen Winkel ungleich 90° ein. Der Winkel kann beispielsweise zwischen 40° und 85° liegen.

Die Druckplatte kann eine Manipulationsöffnung aufweisen, so dass im verbundenen Zustand der Kopf der Knochenschraube durch die Manipulationsöffnung hindurch zugänglich ist. Durch eine solche Manipulationsöffnung wird es möglich, die Druckplatte bereits mit der Knochenplatte zu verbinden, bevor die Knochenschraube in ihre endgültige Position relativ zu der Knochenplatte gebracht ist. Noch nach dem Anbringen der Druckplatte kann durch die Manipulationsöffnung hindurch an der Knochenschraube angegriffen werden, beispielsweise um die Position des mit der Knochenschraube verbundenen Knochenteils zu verändern. Wenn die Knochenschraube ihre endgültige Position relativ zu der Knochenplatte eingenommen hat, kann die Druckplatte gegen die Knochenplatte gespannt werden, so dass der Kopf der Knochenschraube zwischen der Druckplatte und der Knochenplatte fixiert ist.

Das Durchgangsloch hat in einer Richtung einen Durchmesser, der kleiner ist als der Durchmesser des Schraubenkopfs, so dass der Schraubenkopf von der Sitzfläche des Durchgangslochs aufgenommen wird. Vorzugsweise ist die Sitzfläche so gestaltet, dass der Schraubenkopf in der betreffenden Richtung eine definierte Position relativ zu der Knochenplatte hat. Das Durchgangsloch kann so gestaltet sein, dass die Knochenschraube auch in anderen Richtungen nicht relativ zu der Knochenplatte verschoben werden kann. Die Position des Schraubenkopfs in der Knochenplatte ist dann eindeutig bestimmt und es kann lediglich die Ausrichtung des Schafts variiert werden.

Möglich ist es auch, dass das Durchgangsloch in einer Richtung einen Durchmesser hat, der größer ist als der Durchmesser des Schraubenkopfs. In dieser Richtung kann die Knochenplatte relativ zu der Knochenschraube verschoben werden. Dies kann beispielsweise genutzt werden, um nach dem Einschrauben der Knochenschraube die betreffenden Knochenteile in die richtige Position zueinander zu bringen.

Wenn das Durchgangsloch in einer Richtung einen größeren Durchmesser hat als der Schraubenkopf, kann die Sitzfläche nach Art einer Schiene ausgebildet sein, entlang derer der Schraubenkopf kontinuierlich verschoben werden kann und beliebige Zwischenpositionen einnehmen kann. In anderen Ausführungsformen kann die Sitzfläche mit Vorsprüngen versehen sein, die eine Mehrzahl von bevorzugten Positionen für den Schraubenkopf definieren.

Die Manipulationsöffnung ist vorzugsweise ebenfalls so gestaltet, dass sie in einer Richtung einen Durchmesser hat, der kleiner ist als der Durchmesser des Schraubenkopfs. Die an die Manipulationsöffnung angrenzenden Bereiche der Druckplatte können dann Druck auf den Schraubenkopf ausüben, um die Knochenschraube in der gewünschten Stellung zu fixieren. Vorzugsweise stimmt diese Richtung mit der Richtung überein, in der das Durchgangsloch seinen kleinsten Durchmesser hat, wenn die Druckplatte mit der Knochenplatte verbunden ist.

In einer anderen Richtung kann die Manipulationsöffnung einen Durchmesser haben, der größer ist als der Durchmesser des Schraubenkopfs. Es kann dann durch die Manipulationsöffnung hindurch an dem Schraubenkopf angegriffen werden, um die Knochenschraube relativ zu der Knochenplatte zu verschieben. Die Manipulationsöffnung kann ebenfalls nach Art einer Schiene gestaltet sein, entlang derer der Schraubenkopf kontinuierlich verschoben werden kann. Alternativ kann die Manipulationsöffnung mit Vorsprüngen versehen sein, die eine Mehrzahl von bevorzugten Positionen für die Knochenschraube definieren.

Der kleinste Durchmesser der Manipulationsöffnung sollte größer sein als der Durchmesser des Werkzeugs, mit dem an der Knochenschraube angegriffen wird. Das Werkzeug kann dann durch die Manipulationsöffnung hindurchgeführt werden, um in den Schraubenkopf einzugreifen. Hat die Knochenschraube in dem Schraubenkopf eine Ausnehmung (beispielsweise in Form eines Innensechskant-Profils), die für den Eingriff eines Werkzeugs ausgelegt ist, so ist der Durchmesser der Manipulationsöffnung vorzugsweise größer als der Durchmesser der Ausnehmung.

Im verbundenen Zustand ist die Druckplatte mit der Knochenplatte verbunden, so dass der Kopf der Knochenschraube zwischen der Druckplatte und der Knochenplatte eingeschlossen ist. Die Verbindung ist so gestaltet, dass die Druckplatte gegen den Kopf der Knochenschraube gespannt werden kann. Vor dem Spannen der Druckplatte ist der Kopf der Knochenschraube zwar schon zwischen der Knochenplatte und der Druckplatte eingeschlossen, die Knochenschraube kann jedoch noch relativ zu der Knochenplatte und der Druckplatte verschoben werden.

Die zwei Befestigungsschrauben der Schraubverbindung zwischen der Druckplatte und der Knochenplatte können so angeordnet sein, dass sie die Manipulationsöffnung zwischen sich einschließen.

Damit die Druckplatte dem Schraubenkopf einen sicheren Halt vermitteln kann, können Maßnahmen vorgesehen sein, mit denen die Reibung zwischen dem Schraubenkopf und der Druckplatte erhöht wird. Beispielsweise kann der Schraubenkopf in dem Kontaktbereich, in dem die Druckplatte an dem Schraubenkopf anliegt, mit einer Oberflächenstrukturierung versehen sein. Die Oberflächenstrukturierung kann sich um die Ausnehmung des Schraubenkopfs herum erstrecken, in die mit dem Werkzeug eingegriffen wird. Beispielsweise kann von dieser Kante eine Mehrzahl von Vorsprüngen ausgebildet sein, die in Axialrichtung der Knochenschraube spitz zulaufen.

Weiter verbessert werden kann der Halt, wenn die Druckplatte aus einem weicheren Material besteht als die Knochenschraube. Die Druckplatte kann sich dann unter dem Druck des Schraubenkopfs verformen, so dass es zu einem verbesserten Eingriff zwischen dem Schraubenkopf und der Druckplatte kommt. Wenn die Strukturierung am Kopf der Knochenschraube in das Material der Druckplatte eingreift, kann es sogar zu einer formschlüssigen Verbindung kommen. Die Knochenschraube kann beispielsweise aus einer Titanlegierung wie TiAl6V4 bestehen. Die Druckplatte kann aus Reintitan bestehen, vorzugsweise vom Grade 0, Grade 1, Grade 2 oder Grade 3.

Wenn die Druckplatte aus einem weicheren Material besteht als die Knochenplatte, ist dies von Vorteil, um die Druckplatte mit den Befestigungsschrauben unter Spannung setzen zu können. Die Druckplatte hat dann eine erhöhte Elastizität und kann unter eine Biegespannung und unter eine Zugspannung gesetzt werden, ohne dass die Knochenplatte zu großen Kräften ausgesetzt wird. Insbesondere wenn auf diese Weise die Elastizität der Druckplatte erhöht wird, kann die Befestigungsschraube auch einen größeren Winkel mit einer Tangentialebene einschließen, die im Bereich der Bohrung an die Knochenplatte angelegt wird. Beispielsweise kann der Winkel zwischen 5° und 60°, vorzugsweise zwischen 30° und 50° liegen.

Die Druckplatte kann insgesamt aus dem weicheren Material bestehen. Alternativ ist es auch möglich, dass die Druckplatte einen Druckplattenkörper und ein Inlay umfasst, wobei nur das Inlay aus einem weicheren Material besteht. Vorzugsweise bildet das Inlay den Kontaktbereich, mit dem die Druckplatte an dem Schraubenkopf anliegt. Durch den Druckplattenkörper erhält die Druckplatte eine höhere Stabilität, als wenn sie insgesamt aus dem weicheren Material besteht. Das Inlay kann wie beschrieben aus Reintitan bestehen. Der Druckplattenkörper kann beispielsweise aus der Titanlegierung TiAl4V6 bestehen.

In einer bevorzugten Ausführungsform umfasst das Knochenplatten-System mehr als eine erfindungsgemäße Druckplatte, wobei jede Druckplatte einem Durchgangsloch der Knochenplatte zugeordnet ist. Die Knochenplatte kann ein oder mehrere weitere Durchgangslöcher aufweisen, denen keine Druckplatte zugeordnet ist.

Für die Winkelvariabilität zwischen der Knochenschraube und der Knochenplatte ist es von Vorteil, wenn die Fläche des Schraubenkopfs, die auf der Sitzfläche des Durchgangslochs aufliegt, etwa halbkugelförmig geformt ist. Vorzugsweise hat die Sitzfläche des Durchgangslochs im Querschnitt betrachtet eine dazu passende Halbkugelform. Im Rahmen der Erfindung ist es von Vorteil, wenn der Winkel zwischen der Knochenschraube und der Knochenplatte kontinuierlich variiert werden kann, also nicht lediglich eine Auswahl zwischen einer Mehrzahl vorgegebener Richtungen möglich ist.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine schematische Darstellung eines Anwendungsbeispiels eines erfindungsgemäßen KnochenplattenSystems;
- Fig. 2:: die Komponenten eines erfindungsgemäßen Knochenplatten-Systems;
- Fig. 3:: eine alternative Ausführungsform einer erfindungsgemäßen Knochenplatte;
- Fig. 4:: eine schematische Schnittdarstellung eines erfindungsgemäßen Knochenplatten-Systems;
- Fig. 5:: eine Querschnittsansicht eines erfindungsgemäßen Knochenplatten-Systems im verbundenen Zustand;
- Fig. 6:: eine Ansicht von oben auf den Kopf einer erfindungsgemäßen Knochenschraube;
- Fig. 7:: eine alternative Ausführungsform eines erfindungsgemäßen Knochenplatten-Systems; und
- Fig. 8:: das Knochenplatten-System aus Fig. 7 im zusammengesetzten Zustand.

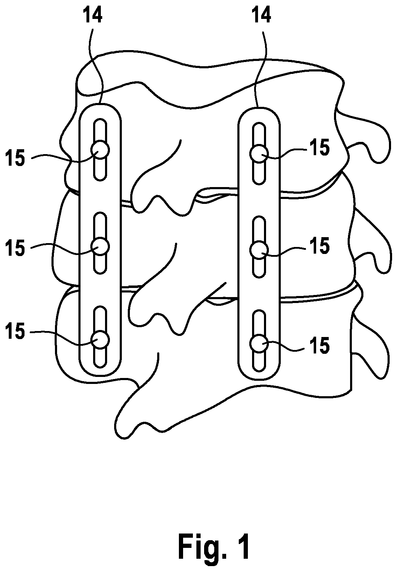

In Fig. 1 ist ein drei Wirbelkörper umfassender Ausschnitt einer menschlichen Wirbelsäule in einer Ansicht von schräg dorsal gezeigt. An dem mittleren Wirbelkörper ist es infolge eines Unfalls zu einer Fraktur gekommen. Mit zwei Knochenplatten 14 sind der obere und der untere Wirbelkörper so relativ zueinander fixiert, so dass der mittlere Wirbelkörper entlastet ist und in Ruhe ausheilen kann. Die Knochenplatte 14 ist mit Knochenschrauben 15, die in die Pedikel des Wirbelkörpers eingeschraubt sind, an den Wirbelkörpern befestigt. Während der Operation liegt der Patient auf dem Bauch. Der Chirurg schafft vom Rücken aus einen Zugang zu den Wirbelkörpern. Diese Art der dorsalen Fixation geht auf Roy Camille zurück.

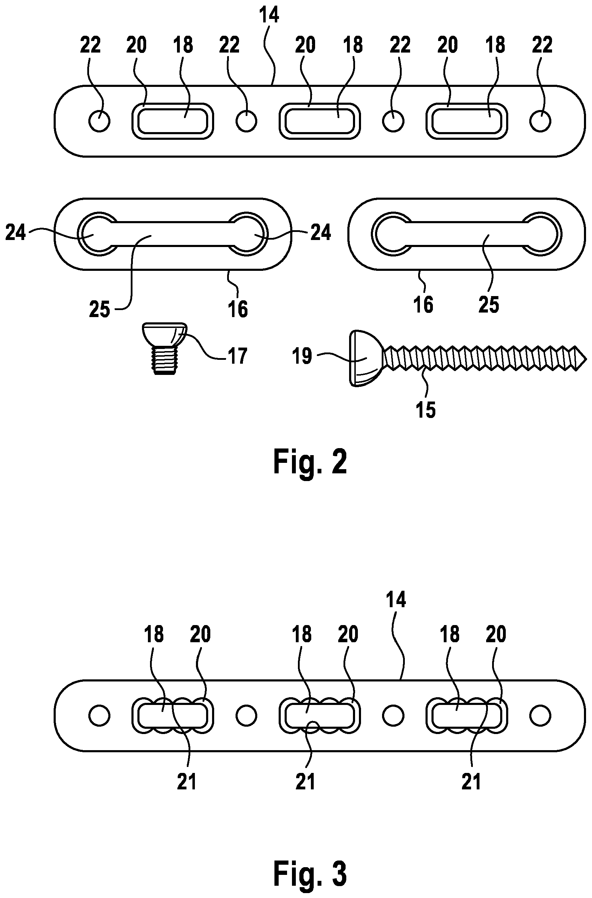

Das erfindungsgemäße Knochenplatten-System umfasst gemäß Fig. 2 eine Knochenplatte 14, zwei Druckplatten 16 sowie eine Mehrzahl von Knochenschrauben 15 und Befestigungsschrauben 17.

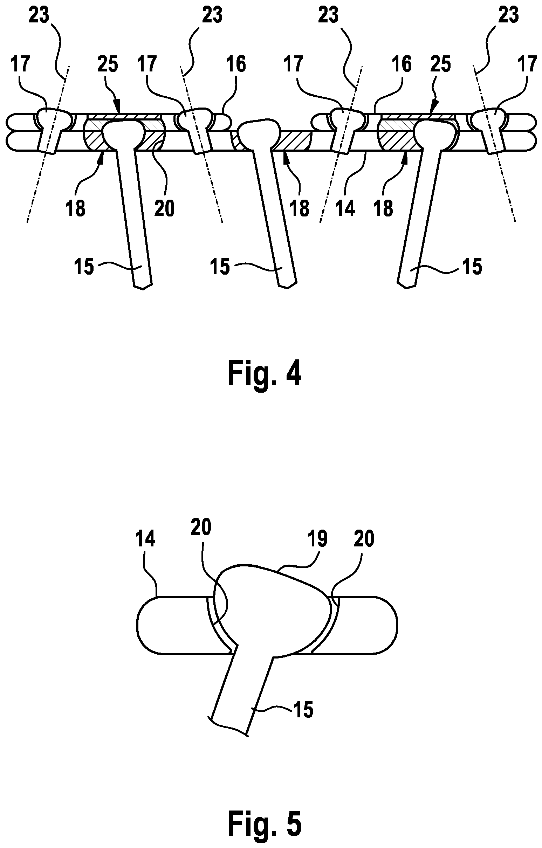

Die Knochenplatte 14 hat drei Durchgangslöcher 18, deren Durchmesser in Querrichtung der Knochenplatte 14 dem Durchmesser des Kopfs 19 der Knochenschraube 15 entspricht. Jedes Durchgangsloch 18 ist mit einer Sitzfläche 20 versehen, die gemäß Fig. 5 im Querschnitt kreissegmentförmig ist, so dass der halbkugelförmige Kopf 19 der Knochenschraube 15 in unterschiedlichen Ausrichtungen der Knochenschraube 15 flächig auf der Sitzfläche 20 aufliegt. Die Sitzfläche 20 kann die Knochenschraube 15 also winkelvariabel aufnehmen. In Querrichtung der Knochenplatte 14 ist die Position der Knochenschraube 15 innerhalb des Durchgangslochs 18 eindeutig bestimmt. In Längsrichtung der Knochenplatte 14 kann die Knochenschraube 15 innerhalb des Durchgangslochs 18 verschoben werden.

Bei der in Fig. 2 gezeigten Ausführungsform sind die Sitzflächen 20 der Durchgangslöcher 18 in Längsrichtung nach Art einer Schiene ausgeführt, entlang derer die Knochenschrauben 15 kontinuierlich verschoben werden können und in beliebigen Zwischenpositionen liegen können. Bei der alternativen Ausführungsform der Fig. 3 sind in der Sitzfläche 20 Vorsprünge 21 ausgebildet, durch die bestimmte bevorzugte Positionen für den Kopf 19 der Knochenschraube 15 definiert werden. Der Kopf 19 der Knochenschraube 15 liegt dann gut an der Sitzfläche 20 an, wenn er zwischen zwei Vorsprüngen 21 positioniert ist.

Die Knochenplatte 14 umfasst außerdem vier Bohrungen 22. Die Bohrungen 22 sind mit einem Innengewinde versehen, das zu dem Gewinde der Befestigungsschrauben 17 passt. Die Bohrungen 22 sind nicht rechtwinklig zur Ebene der Knochenplatte 14 ausgerichtet, sondern die Achsen 23 der Bohrungen 22 sind gemäß Fig. 4 geneigt. Die beiden äußeren Durchgangslöcher 18 der Knochenplatte 14 sind jeweils zwischen zwei Bohrungen 22 eingeschlossen. Bezogen auf das eingeschlossene Durchgangsloch 18 sind die Achsen 23 der Bohrungen 22 gespreizt, der Abstand der Achsen 23 vergrößert sich also in Einschraubrichtung.

Die Druckplatten 16 des erfindungsgemäßen Knochenplatten-Systems haben jeweils zwei Öffnungen 24, deren Abstand an den Abstand der Bohrungen 22 in der Knochenplatte 14 angepasst ist. Wenn die Druckplatte 16 auf die Knochenschraube 15 aufgelegt wird, können die Befestigungsschrauben 17 durch die Öffnungen 24 hindurchgeführt werden und die Befestigungsschrauben 17 in die Bohrungen 22 eingeschraubt werden. Dadurch wird die Druckplatte 16 gespannt und gegen die Knochenplatte 14 gedrückt. Aufgrund der Spreizung der Bohrungen 22 wird die Druckplatte 16 einer Biegespannung sowie einer Zugspannung in Längsrichtung der Knochenplatte 14 ausgesetzt.

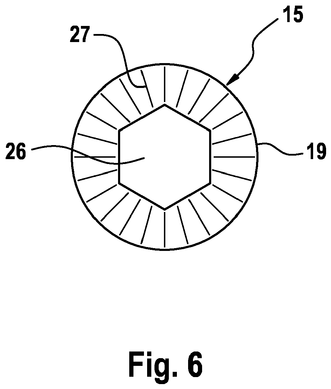

Zwischen den beiden Öffnungen 24, die für die Befestigungsschrauben 17 bestimmt sind, erstreckt sich eine Manipulationsöffnung 25, wobei die Manipulationsöffnung 25 in dem vorliegenden Ausführungsbeispiel eine ununterbrochene Verbindung zwischen den beiden Öffnungen 24 herstellt. In Querrichtung betrachtet ist der Durchmesser der Manipulationsöffnung 25 etwas größer als der Durchmesser der in Fig. 6 gezeigten Innensechskant-Ausnehmung 26 im Kopf 19 der Knochenschraube 15. Es kann also ein zu der Ausnehmung 26 passender Schraubendreher durch die Manipulationsöffnung 25 hindurchgeführt werden.

Bei der Verwendung des erfindungsgemäßen Knochenplatten-Systems im Rahmen der in Fig. 1 gezeigten Operation wird die Knochenplatte 14 auf die Wirbelkörper aufgelegt und die Knochenschrauben 15 werden in Bohrungen des Wirbelkörpers eingeschraubt. Die Knochenschraube 15 wird im ersten Schritt so weit eingeschraubt, dass der Kopf 19 der Knochenschraube 15 in den Bereich des Durchgangslochs 18 kommt. Die Knochenschraube 15 wird jedoch noch nicht so stark angezogen, dass sie die Knochenplatte 14 gegen den Wirbelkörper spannt.

Die Druckplatte 16 wird dann so auf die Knochenplatte 14 aufgelegt, dass der Kopf 19 der Knochenschraube 15 zwischen der Druckplatte 16 und der Knochenplatte 14 eingeschlossen ist. Mit den Befestigungsschrauben 17 wird die Druckplatte 16 an der Knochenplatte 14 befestigt. Auch die Befestigungsschrauben 17 werden im ersten Schritt noch nicht so fest angezogen, dass der Kopf 19 der Knochenschraube 15 eingeklemmt ist. Das Knochenplatten-System ist dann in einem Zustand, in dem die Knochenschraube 15 noch relativ zu dem Durchgangsloch 18 verschoben werden kann.

Mit einem Schraubendreher kann nun durch die Manipulationsöffnung 25 hindurch in die Innensechskant-Ausnehmung 26 der Knochenschraube 15 eingegriffen werden. Wenn in die beiden außen angeordneten Knochenschrauben 15 jeweils ein Schraubendreher eingeführt ist, kann mit einer Spreizzange an den Schraubendrehern angegriffen werden, so dass die äußeren Wirbelkörper voneinander gespreizt werden und der in der Mitte liegende Wirbelkörper entlastet wird. Außerdem kann der Winkel zwischen der Knochenschraube und der Knochenplatte geändert werden, um die Wirbelkörper richtig zueinander auszurichten.

Sind die Wirbelkörper auf diese Weise in die gewünschte Position gebracht, können die Knochenschrauben mit dem Schraubendreher festgezogen werden, so dass die Knochenplatte gegen die Wirbelkörper gespannt wird. Anschließend werden die Befestigungsschrauben fest gezogen, so dass der Kopf der Knochenschraube zwischen der Druckplatte und der Knochenplatte eingeklemmt ist. Die Knochenschraube ist dann winkelstabil mit der Knochenplatte verbunden, was zur Folge hat, dass die Verbindung zwischen der Knochenschraube und dem Wirbelkörper entlastet wird. Das Knochenmaterial ist also geringeren Belastungen ausgesetzt, als wenn die Knochenschraube relativ zu der Knochenplatte beweglich wäre.

Die Druckplatte 16 besteht aus Reintitan Grade 1 und ist damit weicher als die Knochenschraube, die aus TiAl6V4 besteht. Das Material der Druckplatte 16 verformt sich, wenn die Druckplatte gegen den Kopf 19 der Knochenschraube 15 gespannt wird. Der Kopf der Knochenschraube 15 ist in dem Kontaktbereich, in dem die Druckplatte 16 auf der Knochenschraube 15 aufliegt, mit einer Oberflächenstruktur 27 versehen, die eine Vielzahl von radial ausgerichteten Erhöhungen und Vertiefungen aufweist. Die Erhöhungen können in das Material der Druckplatte 16 eindringen, so dass es zu einem Formschluss zwischen der Oberflächenstruktur 27 und der Druckplatte 16 kommt.

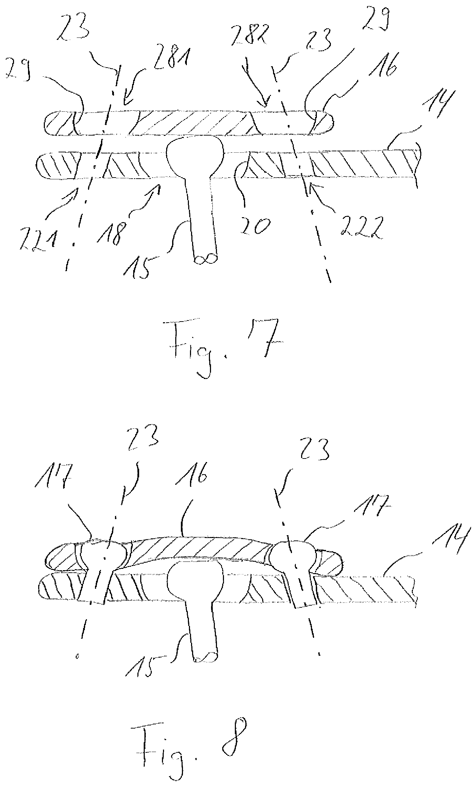

Bei der alternativen Ausführungsform der Figuren 7 und 8 hat die Druckplatte 16 keine Manipulationsöffnung. Die Knochenschraube 15 wird bei diesem Ausführungsbeispiel zunächst bis in ihre endgültige Position festgezogen, bevor die Druckplatte 16 mit der Knochenplatte 14 verbunden wird. Die Knochenschraube 15 ragt in diesem Zustand über die Oberfläche der Knochenplatte 14 hinaus, so dass die Druckplatte 16, die auf die Knochenschraube 15 aufgelegt wird, einen Abstand zu der Knochenplatte 14 hat. Dieser Zustand, in dem die Druckplatte 16 auf die Knochenschraube 15 aufgelegt ist, jedoch noch nicht mit der Knochenplatte 14 verbunden ist, ist in Fig. 7 dargestellt.

Die Achse 23, entlang derer die Befestigungsschraube 17 mit dem Innengewinde der Bohrung 221, 222 in Eingriff gebracht wird, erstreckt sich mittig durch die Aufnahmeöffnung 281, 282 der Druckplatte 16. Da beide Aufnahmeöffnungen 281, 282 mit jeweils einer Bohrung 221, 222 fluchten, kann die Schraubverbindung zwischen der Druckplatte 16 und der Knochenplatte 14 leicht hergestellt werden, ohne dass eines der Elemente unter Spannung steht. Würde die Druckplatte 16 flächig auf der Knochenplatte 14 aufliegen, würden die Aufnahmeöffnungen 281, 282 nicht mit den Bohrungen 221, 222 fluchten.

Die Spannung entsteht erst, wenn die Befestigungsschrauben 17 fest angezogen werden, so dass die äußeren Enden der Druckplatte 16 gegen die Knochenplatte 14 gezogen werden. Die Druckplatte 16 wird dadurch sowohl einer Biegespannung als auch einer Zugspannung in Längsrichtung ausgesetzt. Dies führt dazu, dass die Knochenschraube 15 zuverlässig fixiert ist.

Beim Anziehen verändert sich der Winkel zwischen dem Kopf der Befestigungsschraube 17 und der Aufnahmeöffnung 28. Die Sitzfläche 29 der Aufnahmeöffnung 28 hat eine sphärische Form, so dass die halbkugelförmige Unterseite des Kopfes der Befestigungsschraube 17 auf der Sitzfläche 29 gleiten kann. Unnötige Spannungen zwischen der Befestigungsschraube 17 und der Druckplatte 16 werden dadurch vermieden.

## Patentansprüche

1. Knochenplatten-System, umfassend eine Knochenplatte (14) mit einem Durchgangsloch (18), eine Druckplatte (16) sowie eine Knochenschraube (15), wobei das Durchgangsloch (18) eine winkelvariable Sitzfläche (20) für die Knochenschraube (15) aufweist, und wobei in einem verbundenen Zustand des Systems die Knochenschraube (15) in das Durchgangsloch (18) eingesetzt ist und ein Kopf (19) der Knochenschraube (15) zwischen der Knochenplatte (14) und der Druckplatte (16) eingeschlossen ist, wobei eine zwei Befestigungsschrauben (17) umfassende Schraubverbindung (17, 22) zum Befestigen der Druckplatte (16) an der Knochenplatte (14) vorgesehen ist, **dadurch gekennzeichnet, dass** die Achsen (23) der Befestigungsschrauben (17) gespreizt sind, so dass der Abstand zwischen den Achsen (23) der Befestigungsschrauben (17) in Einschraubrichtung größer wird.

2. Knochenplatten-System nach Anspruch 1, **dadurch gekennzeichnet, dass** die Druckplatte (16) ein erstes Aufnahmeloch (281) und ein zweites Aufnahmeloch (282) für die Befestigungsschrauben (17) aufweist, dass die Knochenplatte (14) eine erste Bohrung (221) und eine zweite Bohrung (221) für die Befestigungsschrauben (17) aufweist, wobei das erste Aufnahmeloch (281) mit der ersten Bohrung (221) fluchtet und das zweite Aufnahmeloch (282) mit der zweiten Bohrung (222) fluchtet, wenn die Druckplatte (16) einen Abstand zu der Knochenplatte (14) hat.

3. Knochenplatten-System nach Anspruch 1 oder 2, **dadurch gekennzeichnet, dass** das erste Aufnahmeloch (281) und/oder das zweite Aufnahmeloch (282) eine winkelvariable Sitzfläche (29) für die die Befestigungsschraube (17) hat.

4. Knochenplatten-System nach einem der Ansprüche 1 bis 3, **dadurch gekennzeichnet, dass** die Druckplatte (16) eine Manipulationsöffnung (25) aufweist, so dass im verbundenen Zustand der Kopf (19) der Knochenschraube (15) durch die Manipulationsöffnung (25) hindurch zugänglich ist.

5. Knochenplatten-System nach Anspruch 4, **dadurch gekennzeichnet, dass** die Aufnahmelöcher (281, 282) so angeordnet sind, dass sie die Manipulationsöffnung (25) zwischen sich einschließen.

6. Knochenplatten-System nach einem der Ansprüche 1 bis 5, **dadurch gekennzeichnet, dass** das Durchgangsloch (18) in einer Richtung einen Durchmesser hat, der größer ist als der Durchmesser des Kopfs (19) der Knochenschraube (15).

7. Knochenplatten-System nach Anspruch 6, **dadurch gekennzeichnet, dass** die Sitzfläche (20) des Durchgangslochs (18) nach Art einer Schiene ausgebildet ist.

8. Knochenplatten-System nach Anspruch 6 oder 7, **dadurch gekennzeichnet, dass** die Sitzfläche (20) des Durchgangslochs (18) mit Vorsprüngen (21) versehen ist und dass die Vorsprünge (21) eine Mehrzahl von bevorzugten Positionen für den Kopf (19) der Knochenschraube (15) definieren.

9. Knochenplatten-System nach einem der Ansprüche 1 bis 8, **dadurch gekennzeichnet, dass** die Manipulationsöffnung (25) in einer Richtung einen Durchmesser hat, der größer ist als der Durchmesser des Kopfs (19) der Knochenschraube (15).

10. Knochenplatten-System nach einem der Ansprüche 1 bis 9, **dadurch gekennzeichnet, dass** der kleinste Durchmesser der Manipulationsöffnung (25) größer ist als der Durchmesser eines für die Knochenschraube (15) vorgesehenen Werkzeugs.

11. Knochenplatten-System nach einem der Ansprüche 1 bis 10, **dadurch gekennzeichnet, dass** der Kopf (19) der Knochenschraube (15) im Kontaktbereich zu der Druckplatte (16) mit einer Oberflächenstrukturierung (27) versehen ist.

12. Knochenplatten-System nach einem der Ansprüche 1 bis 11, **dadurch gekennzeichnet, dass** das Material der Druckplatte (16) weicher ist als das Material der Knochenschraube (15).

13. Knochenplatten-System nach Anspruch 12, **dadurch gekennzeichnet, dass** die Druckplatte (16) aus Reintitan besteht.

14. Knochenplatten-System nach Anspruch 13, **dadurch gekennzeichnet, dass** die Druckplatte (16) aus Reintitan Grade 0, Reintitan Grade 1, Reintitan Grade 2 oder Reintitan Grade 3 besteht.

15. Knochenplatten-System nach einem der Ansprüche 1 bis 14, **dadurch gekennzeichnet, dass** die Druckplatte (16) einen Druckplattenkörper und ein Inlay umfasst, wobei das Inlay aus einem weicheren Material besteht als der Druckplattenkörper.

## Claims

1. A bone plate system, comprising a bone plate (14) with a through-hole (18), a pressure plate (16), and a bone screw (15), wherein the through-hole (18) has a seat surface (20) with a variable angle for the bone screw (15), and wherein, in a connected state of the system, the bone screw (15) is inserted into the through-hole (18) and a head (19) of the bone screw (15) is enclosed between the bone plate (14) and the pressure plate (16), wherein a screw connection (17, 22) comprising two fastening screws (17) is provided for fastening the pressure plate (16) to the bone plate (14), **characterized in that** the axes (23) of the fastening screws (17) are spread apart such that the distance between the axes (23) of the fastening screws (17) increases in the screwing-in direction.

2. The bone plate system as claimed in claim 1, **characterized in that** the pressure plate (16) has a first receiving hole (281) and a second receiving hole (282) for the fastening screws (17), **in that** the bone plate (14) has a first bore (221) and a second bore (221) for the fastening screws (17), wherein the first receiving hole (281) is flush with the first bore (221) and the second receiving hole (282) is flush with the second bore (222) when the pressure plate (16) is at a distance from the bone plate (14).

3. The bone plate system as claimed in claim 1 or 2, **characterized in that** the first receiving hole (281) and/or the second receiving hole (282) has a seat surface (29) with a variable angle for the fastening screw (17).

4. The bone plate system as claimed in one of claims 1 through 3, **characterized in that** the pressure plate (16) has a manipulation opening (25) such that, in the connected state, the head (19) of the bone screw (15) is accessible through the manipulation opening (25).

5. The bone plate system as claimed in claim 4, **characterized in that** the receiving holes (281, 282) are arranged such that they enclose the manipulation opening (25) between themselves.

6. The bone plate system as claimed in one of claims 1 through 5, **characterized in that** the through-hole (18) has, in one direction, a diameter that is greater than the diameter of the head (19) of the bone screw (15).

7. The bone plate system as claimed in claim 6, **characterized in that** the seat surface (20) of the through-hole (18) is designed in the manner of a rail.

8. The bone plate system as claimed in claim 6 or 7, **characterized in that** the seat surface (20) of the through-hole (18) is provided with projections (21), and **in that** the projections (21) define a plurality of preferred positions for the head (19) of the bone screw (15).

9. The bone plate system as claimed in one of claims 1 through 8, **characterized in that** the manipulation opening (25) has, in one direction, a diameter that is greater than the diameter of the head (19) of the bone screw (15).

10. The bone plate system as claimed in one of claims 1 through 9, **characterized in that** the smallest diameter of the manipulation opening (25) is greater than the diameter of a tool provided for the bone screw (15).

11. The bone plate system as claimed in one of claims 1 through 10, **characterized in that** the head (19) of the bone screw (15) is provided with surface structuring (27) in the area of contact with the pressure plate (16).

12. The bone plate system as claimed in one of claims 1 through 11, **characterized in that** the material of the pressure plate (16) is softer than the material of the bone screw (15).

13. The bone plate system as claimed in claim 12, **characterized in that** the pressure plate (16) is made of pure titanium.

14. The bone plate system as claimed in claim 13, **characterized in that** the pressure plate (16) is made of pure titanium grade 0, pure titanium grade 1, pure titanium grade 2 or pure titanium grade 3.

15. The bone plate system as claimed in one of claims 1 through 14, **characterized in that** the pressure plate (16) comprises a pressure plate body and an inlay, wherein the inlay is made of a softer material than the pressure plate body.

## Revendications

1. Système de plaques d'ostéosynthèse, incluant une plaque d'ostéosynthèse (14) avec un trou traversant (18), une plaque de pression (16) ainsi qu'une vis à os (15), dans lequel le trou traversant (18) comporte une surface d'assise (20) d'angle variable pour la vis à os (15) et dans lequel, dans un état relié du système, la vis à os (15) est mise en place dans le trou traversant (18) et une tête (19) de la vis à os (15) est enfermée entre la plaque d'ostéosynthèse (14) et la plaque de pression (16), dans lequel il est prévu une liaison vissée (17, 22) incluant de vis de fixation (17) pour fixer la plaque de pression (16) sur la plaque d'ostéosynthèse (14), **caractérisé en ce que** les axes (23) des vis de fixation (17) sont écartés, de sorte que la distance entre les axes (23) des vis de fixation (17) augmente dans la direction de vissage.

2. Système de plaques d'ostéosynthèse selon la revendication 1, **caractérisé en ce que** la plaque de pression (16) comprend un premier trou de réception (281) et un second trou de réception (282) pour les vis de fixation (17), **en ce que** la plaque d'ostéosynthèse (14) comporte un premier perçage (221) et un second perçage (222) pour les vis de fixation (17), de sorte que le premier trou de réception (281) est aligné avec le premier perçage (221) et le second trou de réception (282) est aligné avec le second perçage (22) quand la plaque de pression (16) est à distance de la plaque d'ostéosynthèse (14).

3. Système de plaques d'ostéosynthèse selon la revendication 1 ou 2, **caractérisé en ce que** le premier trou de réception (281) est/ou le second trou de réception (282) comporte une surface d'assise (29) d'angle variable pour la vis de fixation (17).

4. Système de plaques d'ostéosynthèse selon l'une des revendications 1 à 3, **caractérisé en ce que** la plaque de pression (16) comporte une ouverture de manipulation (25) de sorte que, dans l'état relié, la tête (19) de la vis d'ostéosynthèse (15) est accessible à travers l'ouverture de manipulation (25).

5. Système de plaques d'ostéosynthèse selon la revendication 4, **caractérisé en ce que** les trous de réception (281, 282) sont agencés de telle façon qu'ils enferment entre eux l'ouverture de manipulation (25).

6. Système de plaques d'ostéosynthèse selon l'une des revendications 1 à 5, **caractérisé en ce que** le trou traversant (18) présente dans une direction un diamètre qui est plus grand que le diamètre de la tête (19) de la plaque d'ostéosynthèse (15).

7. Système de plaques d'ostéosynthèse selon la revendication 6, **caractérisé en ce que** la surface d'assise (20) du trou traversant (18) est réalisée à la manière d'un rail.

8. Système de plaques d'ostéosynthèse selon la revendication 6 ou 7, **caractérisé en ce que** la surface d'assise (20) du trou traversant (18) est dotée de saillies (21), et **en ce que** les saillies (21) définissent une pluralité de positions préférées pour la tête (19) de la vis à os (15).

9. Système de plaques d'ostéosynthèse selon l'une des revendications 1 à 8, **caractérisé en ce que** l'ouverture de manipulation (25) présente dans une direction un diamètre qui est plus grand que le diamètre de la tête (19) de la vis à os (15).

10. Système de plaques d'ostéosynthèse selon l'une des revendications 1 à 9, **caractérisé en ce que** le plus petit diamètre de l'ouverture de manipulation (25) est plus grand que le diamètre d'un outil prévu pour la vis à os (15).

11. Système de plaques d'ostéosynthèse selon l'une des revendications 1 à 10, **caractérisé en ce que** la tête (19) de la vis à os (15) est dotée d'une structure de surface (27) dans la zone de contact avec la plaque de pression (16).

12. Système de plaques d'ostéosynthèse selon l'une des revendications 1 à 11, **caractérisé en ce que** le matériau de la plaque de pression (16) est plus souple que le matériau de la vis à os (15).

13. Système de plaques d'ostéosynthèse selon la revendication 12, **caractérisé en ce que** la plaque de pression (16) est en titane pur.

14. Système de plaques d'ostéosynthèse selon la revendication 13, **caractérisé en ce que** la plaque de pression (16) est en titane pur de nuance 0, en titane pur de nuance 1, en titane pur de nuance 2 ou en titane pur de nuance 3.

15. Système de plaques d'ostéosynthèse selon l'une des revendications 1 à 14, **caractérisé en ce que** la plaque de pression (16) inclut un corps et un insert, et l'insert est en un matériau plus souple que le corps de la plaque de pression.
